# EUROPEAN PATENT SPECIFICATION

(11) **EP 4 570 181 B1**
(45) Date of publication and mention of the grant of the patent: **06.05.2026**
(21) Application number: 23216699.1
(22) Date of filing: 14.12.2023
(51) Int. Cl.: A61B 6/12, A61B 6/00, A61M 25/10

(54) **DETERMINING AN INFLATION DURATION OF A BALLOON CATHETER**
BESTIMMUNG EINER AUFBLASDAUER EINES BALLONKATHETERS
DÉTERMINATION D'UNE DURÉE DE GONFLAGE D'UN CATHÉTER À BALLONNET

(43) Date of publication of application: 18.06.2025
(73) Proprietor: Siemens Healthineers AG, 91301 Forchheim (DE)
(72) Inventor: RADHAKRISHNA, Pooja, 560079 Bangalore (IN)
(74) Representative: Siemens Healthineers Patent Attorneys

(56) References cited:
- WO-A1-2023/085253
- MORGAN T. HARLOFF: "A step-by-step guide to transseptal valve-in-valve transcatheter mitral valve replacement", ANNALS OF CARDIOTHORACIC SURGERY, vol. 10, no. 1, 1 January 2021 (2021-01-01), pages 113 - 121, XP093159646, ISSN: 2225-319X, DOI: 10.21037/acs-2020-mv-104
- VINCENT BISMUTH: "Image processing algorithms for the visualization of interventional devices in X-ray fluoroscopy", HAL OPEN SCIENCE, 9 January 2012 (2012-01-09), XP093159985, Retrieved from the Internet <URL:https://theses.hal.science/tel-00747682v2/document>

## Description

The present invention is directed to a computer implemented method for determining an inflation duration of a balloon catheter and to a corresponding method for x-ray imaging. The invention is further directed to a data processing apparatus for carrying out such a computer implemented method, to an x-ray imaging device comprising such a data processing apparatus, and to corresponding computer program products.

Balloon angioplasty is a procedure used to open narrowed or blocked vessels, in particular arteries. It uses a balloon catheter that is inserted into the vessel. At the place where deposits of plaque have closed off or narrowed the channel for blood flow, the balloon catheter is inflated. The balloon catheter may in some cases be drug coated or act as a carrier for a stent to be deployed in the vessel depending on the intended therapy. Balloon angioplasty is commonly carried out under x-ray supervision.

The inflation duration of the balloon catheter corresponds to a time period during which it is inflated or stays at its maximum inflation state until it starts to be deflated or to an accordingly cumulated time in case of multiple inflation and deflation phases. The inflation duration is known to be a critical parameter in view of the success of therapy and in view of the risk for unintended damage of the vessel.

Usually, the inflation duration is determined by manually starting and stopping a timer. This is, however, error-prone and potentially leads to a low accuracy of the determined inflation duration.

Patent application WO 2023/085253 A1 discloses an image processing apparatus with the capability to recognize a specific surgical situation.

Scientific publication Morgan T. Harloff: "A step-by-step guide to transseptal valve-in-valve transcatheter mitral valve replacement",Annals of Cardiothoracic Surgery, vol. 10, no. 1, 1 January 2021 (2021-01-01), pages 113-121, XP093159646,ISSN: 2225-319X, 001: 10.21037/acs-2020-mv-104 discloses example images of balloon catheters and implants.

It is an objective of the present invention to increase the accuracy and/or reliability of determining the inflation duration of a balloon catheter, in particular in real-time.

This objective is achieved by the subject matter of the independent claim. Further implementations and preferred embodiments are subject matter of the dependent claims.

The invention is based on the idea to determine and track the diameter of the balloon catheter during x-ray supervision based on the respective x-ray images automatically and to determine the inflation duration based on the tracked diameter, in particular by determining respective starting times of an inflation phase and a deflation phase.

According to an aspect of the invention, a computer implemented method for determining an inflation duration of a balloon catheter is provided. Therein, a sequence of x-ray images depicting the balloon catheter inside a vessel of a patient is received. At least one geometric parameter including a diameter of the balloon catheter is determined based on the sequence of x-ray images and tracked over the sequence of x-ray images. A first inflation starting time of a first inflation phase, during which the diameter is increasing, is determined based on the tracked diameter. A first deflation starting time of a first deflation phase, during which the diameter is decreasing and which lies after the first inflation phase, is determined based on the tracked diameter. The inflation duration is computed depending on a first time difference between the first inflation starting time and the first deflation starting time.

Unless stated otherwise, all steps of the computer-implemented method may be performed by a data processing apparatus, which comprises at least one computing unit. In particular, the at least one computing unit is configured or adapted to perform the steps of the computer-implemented method. For this purpose, the at least one computing unit may for example store a computer program comprising instructions which, when executed by the at least one computing unit, cause the at least one computing unit to execute the computer-implemented method.

For each implementation of the computer implemented method, a corresponding implementation of a method for determining the inflation duration of the balloon catheter, which is not purely computer implemented, is obtained by including respective steps for generating the sequence of X-ray images, in particular using X-ray source and an X-ray detector.

The sequence of X-ray images corresponds, for example, to a sequence of frame intervals, wherein one x-ray image is received for each of the frame intervals. The frame intervals are, in particular, consecutive frame intervals following each other, in particular in a sequence. In particular, the positions of the X-ray source and the X-ray detector with respect to a region of interest containing the vessel and the balloon catheter, is constant during the sequence of frame intervals.

The balloon catheter extends approximately along a longitudinal direction. In a working portion of the balloon catheter along the longitudinal direction, the balloon catheter has an approximately circular cross section perpendicular to the longitudinal direction. The diameter of the balloon catheter can be understood as the diameter of that circle. In an X-ray image, which is a projection image unless stated otherwise, the diameter corresponds to the distance between two lateral edges of the balloon catheter perpendicular to the longitudinal direction, in particular at a longitudinal position corresponding to a working length of the balloon catheter.

The geometric parameters of the balloon catheter may be determined using known methods for image analysis or object recognition including, but not limited to, intensity models, model-based image processing, artificial neural networks, in particular convolutional neural networks or transformer-based networks, for example vision-transformers, filtering methods as detection methods, in particular nonlinear filtering, linear discriminant analysis and/or nonlinear discriminant analysis.

The diameter may be directly obtained using such methods or other geometrical parameters, such as the positions of the lateral edges of the balloon catheter, may be obtained from such methods and the diameter may be calculated based on these other geometrical parameters.

Tracking the at least one geometric parameter over the sequence of X-ray images may be understood such that the at least one geometric parameter is determined for each X-ray image of the sequence or for a subset of the sequence of X-ray images, for example every n-th X-ray image of the sequence with n > 1, for example n > 1 and n < 10.

In particular, tracking the at least one geometric parameter over the sequence of X-ray images includes tracking the diameter over the sequence of X-ray images. By tracking the diameter, the first inflation phase and the first deflation phase may be identified and, consequently, the first inflation starting time and the first deflation starting time may be determined. In particular, the first inflation phase may be defined as a phase wherein the diameter is increasing and the first deflation phase may be defined as a phase wherein the diameter is decreasing, in particular after the first inflation phase. It is noted, however, that in some embodiments additional conditions may apply for identifying the first inflation phase and the first deflation phase, for example a stability or constancy of other geometric parameters of the balloon catheter, such as longitudinal end positions of the balloon catheter, a symmetry of the balloon catheter, and so forth. This holds analogously for other phases of the balloon catheter that may be relevant in further embodiments.

For use cases or use situations which may arise in a computer implemented method according to the invention and which are not explicitly described herein, it may be provided that, in accordance with the method, an error message and/or a prompt for user feedback is output and/or a default setting and/or a predetermined initial state is set. This may include situations where the geometric parameters and/or a phase of the balloon catheter may not be identified reliably.

The first deflation phase lies after the first inflation phase, however, does not necessarily follow directly after the first inflation phase. For example, the first deflation phase may follow directly after the first inflation phase or a stationary phase may lie between the first inflation phase and the first deflation phase.

It is noted that the first inflation starting time and the first deflation starting time are respective time instances rather than time periods. The inflation duration, on the other hand, is a time period.

The inflation duration may be equal to the first time difference. This may for example be the case, if the balloon catheter is inflated and deflated exactly once. On the other hand, in use cases where the balloon catheter is inflated and deflated multiple times, the inflation duration may also be a sum of the first time difference and corresponding further time differences computed analogously until the balloon catheter is for example retracted from the vessel or until the catheter position in the vessel is for example changed.

The diameter of the balloon catheter and its dynamic behavior, in particular its increase or decrease, can be considered as a reliable indicator for identifying the first inflation phase and the first deflation phase. Consequently, by means of the computer implemented method according to the invention, the inflation duration may be automatically determined with an increased reliability and accuracy, in particular in real-time.

According to several embodiments, the first inflation starting time is determined as a time, in particular time instance, of a transition from a first static phase, during which the diameter is essentially constant, to the first inflation phase.

By defining the first inflation starting time as the time of said transition, it is ensured that the balloon catheter is in a stable state when the relevant period for computing the inflation duration starts. This further increases the reliability and the accuracy of the determined inflation duration.

That the diameter is essentially constant may be understood such that it is constant or changes only then a predefined tolerance range. For example, the tolerance range may be given by a predefined minimum diameter difference and a predefined maximum diameter difference. The minimum diameter difference and the maximum diameter difference may be relative differences with respect to the current diameter or absolute differences. Consequently, that the diameter is increasing may be understood such that it is increasing and its change is greater than the predefined tolerance range. Analogously, that the diameter is decreasing may be understood such that it is decreasing and its change is less than the predefined tolerance range.

In particular, the time of the transition from the first static phase to the first inflation phase corresponds to a time instance, prior to which the diameter is essentially constant, and after which the diameter is increasing.

For example, the first inflation phase follows directly after the first static phase.

According to several embodiments, the at least one geometric parameter includes a longitudinal end position of the balloon catheter. The longitudinal end position is determined to be essentially constant during the first static phase.

By ensuring that the longitudinal end position is essentially constant during the first static phase, it is further ensured that the balloon catheter is in a stable state when the relevant period for computing the inflation duration starts. This further increases the reliability and the accuracy of the determined inflation duration.

That the longitudinal end position is essentially constant may be understood such that it is constant or changes only then a predefined tolerance range. For example, the tolerance range may be given by a predefined minimum position difference and a predefined maximum position difference. The minimum position difference and the maximum position difference may be relative differences with respect to the current longitudinal end position or absolute differences. The same holds analogously for a further longitudinal end position of the balloon catheter in respective embodiments.

In some embodiments, the longitudinal end position is determined to be essentially constant during the first inflation phase and/or during the first deflation phase and, if applicable, during the second static phase.

According to several embodiments, the at least one geometric parameter includes a further longitudinal end position of the balloon catheter. The longitudinal end position and the further longitudinal end position lie at opposite longitudinal sides of the balloon catheter. The further longitudinal end position is determined to be essentially constant during the first static phase.

According to several embodiments, the first deflation starting time is determined as a time, in particular time instance, of a transition from the first inflation phase to the first deflation phase.

Therein, in particular, the first deflation phase follows directly after the first inflation phase.

According to several embodiments, the first deflation starting time is determined as a time, in particular time instance, of a transition from a second static phase, during which the diameter is essentially constant and which lies between the first inflation phase and the first deflation phase, to the first deflation phase.

Therein, in particular, the first deflation phase follows directly after the second static phase and the second static phase follows directly after the first inflation phase.

Since the balloon catheter has reached its maximum inflation state and maximum diameter, which remain essentially constant during the second static phase, the effect of the balloon catheter on the vessel during the second static phase may be relevant in view of the success of therapy or in view of the risk for unintended damage of the vessel. This is addressed in said embodiments, since the duration of the second static phase contributes to the inflation duration.

According to several embodiments, a first lateral edge position of the balloon catheter and a second lateral edge position of the balloon catheter are determined based on the sequence of X-ray images and, in particular tracked over the sequence of X-ray images. The diameter is determined as a distance between the first lateral edge position and the second lateral edge position.

The first lateral end position and the second lateral end position can be understood as lateral positions of respective lateral edges of the balloon catheter as depicted in the X-ray images. The first lateral end position and the second lateral end position lie at opposite lateral sides of the balloon catheter in the X-ray images.

According to several embodiments, the at least one geometric parameter includes a lateral center position of the balloon catheter. A first lateral distance between the first lateral edge position and the lateral center position is determined and a second lateral distance between the second lateral edge position and the lateral center position is determined. The first lateral distance and the second lateral distance are determined to be essentially equal during the static phase.

The explanations regarding the diameter and the longitudinal end position being essentially constant hold analogously for the lateral distances being essentially equal to each other.

In some embodiments, first lateral distance and the second lateral distance are determined to be essentially equal during the first inflation phase and/or during the first deflation phase and/or, if applicable, during the second static phase.

By ensuring that the lateral distances are essentially equal to each other during the first static phase, it is further ensured that the balloon catheter is in a stable state when the relevant period for computing the inflation duration starts. This further increases the reliability and the accuracy of the determined inflation duration. The same holds analogously, if applicable, for the first inflation phase and/or the first deflation phase and/or the second static phase.

According to several embodiments, a first marker position of a first radio-opaque marker of the balloon catheter and a second marker position of a second radio-opaque marker of the balloon catheter are determined based on the sequence of x-ray images and, in particular, tracked over the sequence of x-ray images. The lateral center position is determined depending on a straight line connecting the first marker position to the second marker position.

Therein, it is provided that the radio-opaque markers are located in the actual lateral center of the balloon catheter at different longitudinal positions. Consequently, the straight line connecting the radio-opaque markers is a reliably measure for the actual lateral center position.

According to several embodiments, a second inflation starting time of a second inflation phase, during which the diameter is increasing and which lies after the first deflation phase, is determined based on the tracked diameter. A second deflation starting time of a second deflation phase, during which the diameter is decreasing and which lies after the second inflation phase, is determined based on the tracked diameter. The inflation duration is computed depending on a second time difference between the second inflation starting time and the second deflation starting time, for example depending on a sum of the first time difference and the second time difference.

The explanations with respect to the first inflation phase and the first deflation phase carry over analogously to the second inflation phase and the second deflation phase. This includes also further embodiments with further static phases analog to the first static phase and/or the second static phase.

According to a further aspect of the invention, a method for X-ray imaging is provided. Therein, respective X-rays are emitted by an X-ray source for a sequence of frame intervals. A respective detector dataset is generated, in particular by an X-ray detector, for each of the sequence of frame intervals depending on portions of the respective X-rays passing through a region of interest containing the vessel and the balloon catheter during the respective frame interval. The sequence of X-ray images is generated depending on the detector datasets generated for the sequence of frame intervals. A computer implemented method for determining the inflation duration of the balloon catheter according to the invention is carried out based on the sequence of X-ray images.

In particular, exactly one respective X-ray image is generated for each of the frame intervals.

According to a further aspect of the invention, a data processing apparatus comprising at least one computing unit is provided. The at least one computing unit is adapted to carry out a computer implemented method for determining the inflation duration of the balloon catheter according to the invention.

A computing unit may in particular be understood as a data processing device, which comprises processing circuitry. The computing unit can therefore in particular process data to perform computing operations. This may also include operations to perform indexed accesses to a data structure, for example a look-up table, LUT.

In particular, the computing unit may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The computing unit may also include one or more processors, for example one or more microprocessors, one or more central processing units, CPU, one or more graphics processing units, GPU, and/or one or more signal processors, in particular one or more digital signal processors, DSP. The computing unit may also include a physical or a virtual cluster of computers or other of said units.

In various embodiments, the computing unit includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, an X-ray imaging device comprising a data processing apparatus according to the invention, an X-ray source, and an X-ray detector. The at least one computing unit is configured to control the X-ray source to emit respective X-rays for a sequence of frame intervals. The X-ray detector is configured to generate a respective detector dataset for each of the sequence of frame intervals depending on portions of the respective X-rays passing through a region of interest containing the vessel and the balloon catheter during the respective frame interval. The at least one computing unit is configured to generate the sequence of X-ray images depending on the detector datasets generated for the sequence of frame intervals.

The at least one computing unit may, in some embodiments, comprise one or more control units for controlling the X-ray source.

Further implementations of the X-ray imaging device according to the invention follow directly from the various embodiments of the methods according to the invention and vice versa. In particular, individual features and corresponding explanations as well as advantages relating to the various implementations of the methods according to the invention can be transferred analogously to corresponding implementations of the X-ray imaging device according to the invention. In particular, the X-ray imaging device according to the invention is designed or programmed to carry out a method according to the invention. In particular, the X-ray imaging device according to the invention carries out a method according to the invention.

According to a further aspect of the invention, a computer program comprising instructions is provided. When the instructions are executed by a data processing apparatus, the instructions cause the data processing apparatus to carry out a computer implemented method for determining an inflation duration of a balloon catheter according to the invention.

The instructions may be provided as program code, for example. The program code can for example be provided as binary code or assembler and/or as source code of a programming language, for example C, and/or as program script, for example Python.

Related to the invention but not being an element of the invention, a further computer program comprising further instructions can be provided. When the further instructions are executed by an X-ray imaging device according to the invention, in particular by the data processing apparatus of the X-ray imaging device, the further instructions cause the X-ray imaging device to carry out a method for X-ray imaging according to the invention.

The further instructions may be provided as program code, for example. The program code can for example be provided as binary code or assembler and/or as source code of a programming language, for example C, and/or as program script, for example Python.

According to a further aspect of the invention, a computer-readable storage medium, in particular a tangible and/or non-transitory computer-readable storage medium, storing a computer program and/or a further computer program according to the invention is provided.

The computer program, the further computer program and the computer-readable storage medium are respective computer program products comprising the instructions and/or the further instructions.

Further features and feature combinations of the invention are obtained from the figures and their description as well as the claims. In particular, further implementations of the invention may not necessarily contain all features of one of the claims. Further embodiments of the inventions may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows schematically an exemplary implementation of an X-ray imaging device according to the invention;
- FIG 2: shows a schematic flow diagram of an exemplary implementation of a computer implemented method for determining an inflation duration of a balloon catheter according to the invention;
- FIG 3: shows a schematic flow diagram of a medical procedure, which comprises an exemplary implementation of a method for X-ray imaging according to the invention;
- FIG 4: shows schematically a balloon catheter as depicted in an X-ray image;
- FIG 5: shows schematically a geometric parametrization of a balloon catheter as depicted in an X-ray image;
- FIG 6: shows schematically a vessel;
- FIG 7: shows schematically the vessel of FIG 6 and a balloon catheter inside the vessel in a first state;
- FIG 8: shows schematically the vessel of FIG 6 and a balloon catheter inside the vessel in a second state;
- FIG 9: shows schematically the vessel of FIG 6 and a balloon catheter inside the vessel in a third state; and
- FIG 10: shows schematically the vessel of FIG 6 and a balloon catheter inside the vessel in a fourth state.

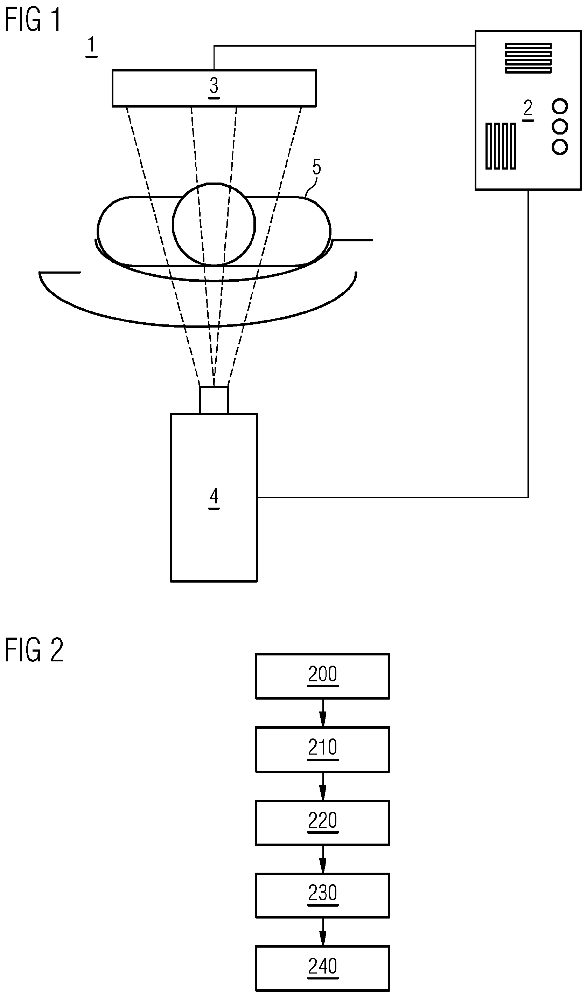

FIG 1 shows schematically an exemplary implementation of an X-ray imaging device 1 according to the invention. The X-ray imaging device 1 comprises a data processing apparatus 2 according to the invention, an X-ray source 4, and an X-ray detector 3. The data processing apparatus 2 comprises at least one computing unit.

The at least one computing unit is configured to control the X-ray source 4 to emit respective X-rays for a sequence of frame intervals. The X-ray detector 3 is configured to generate a respective detector dataset for each of the sequence of frame intervals depending on portions of the respective X-rays passing through a region of interest containing a vessel 19 of a patient 5 and a balloon catheter, 6, which has been inserted into the vessel 19, during the respective frame interval. The at least one computing unit is configured to generate a sequence of X-ray images depending on the detector datasets generated for the sequence of frame intervals.

The at least one computing unit is configured to carry out a computer implemented method for determining an inflation duration of the balloon catheter 6 according to the invention. FIG 2 shows a schematic flow diagram of an exemplary implementation of such a computer implemented method.

The at least one computing unit receives the sequence of X-ray images depicting the balloon catheter 6 inside the vessel 19 of the patient 5 in step 200. In step 210, the at least one computing unit determines at least one geometric parameter including a diameter 18 of the balloon catheter 6 based on the sequence of X-ray images and tracks it over the sequence of X-ray images. The at least one computing unit determines a first inflation starting time of a first inflation phase, during which the diameter 18 is increasing, based on the tracked diameter 18 in step 230. The at least one computing unit determines a first deflation starting time of a first deflation phase, during which the diameter 18 is decreasing and which lies after the first inflation phase, based on the tracked diameter 18 in step 240. The at least one computing unit computes the inflation duration depending on a first time difference between the first inflation starting time and the first deflation starting time in step 240.

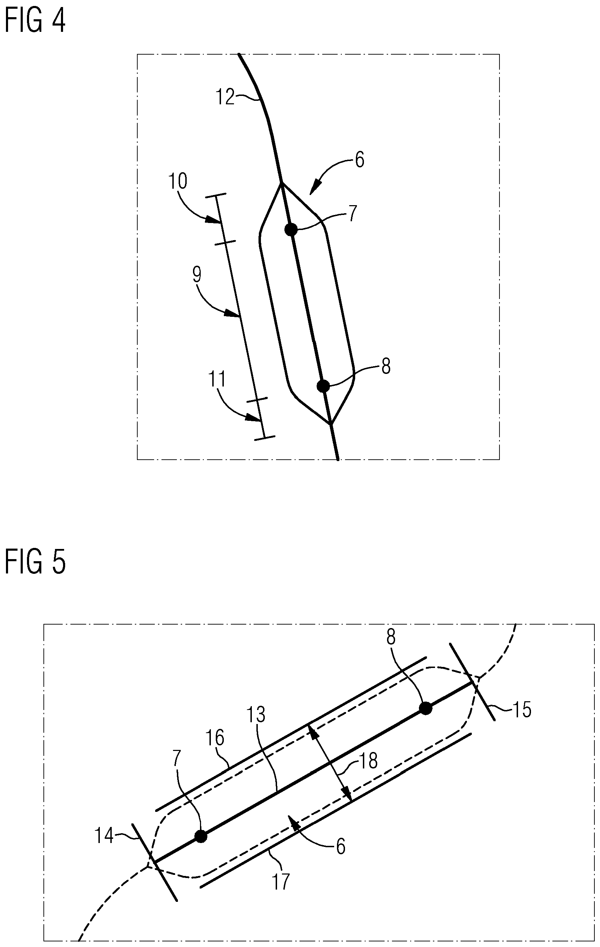

FIG 4 shows schematically a balloon catheter 6 as depicted in an X-ray image. FIG 5 shows schematically a possible geometric parametrization of the balloon catheter 6. The balloon catheter 6 extends essentially along a longitudinal direction and may be operated and moved by means of a guide wire 12. In a longitudinal working range 9, the balloon catheter 6 has an approximately cylindric shape with an approximately circular cross section defining the diameter 18. On both longitudinal sides of the working range 9, the balloon catheter 6 comprises a respective shoulder range 10, 11.

In some embodiments, the balloon catheter 6 comprises two radio-opaque markers 7, 8 located at different longitudinal positions in the lateral center of the working range. A straight line 13, also denoted as baseline 13, connecting the radio-opaque markers 7, 8 in the X-ray images may be used to define a lateral center position of the balloon catheter 6. The positions of the radio-opaque markers 7, 8 and contours of the balloon catheter 6 may be determined in the X-ray images by means of known methods as described earlier. In this way, apart from the lateral center position of the balloon catheter 6, lateral edges 16, 17 and/or longitudinal edge positions 14, 15 of the balloon catheter 6 may be determined. The diameter 18 may be estimated as a perpendicular distance between the lateral edges 16, 17.

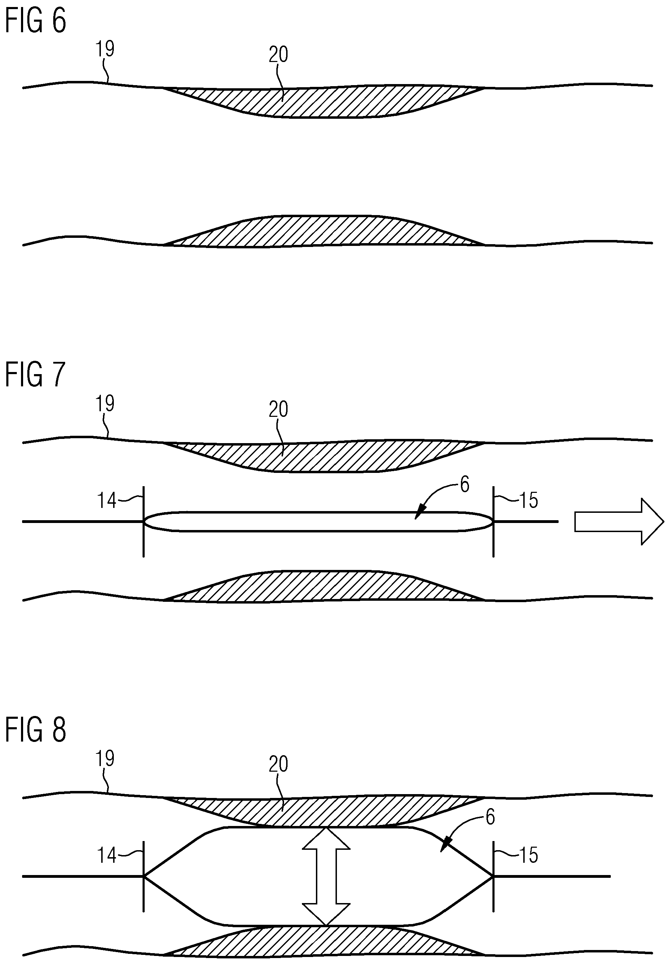

FIG 6 shows a longitudinal section of the vessel 19 and a stenosis 20 formed for example by plaques on the inner walls of the vessel 19. The stenosis 20 may for example be detected by contrast dye injection and image acquisition and/or by optical coherence tomography, OCT, and/or intravascular ultrasound, IVUS.

FIG 7 shows the vessel 19 after the balloon catheter 6 has been inserted into the vessel 19 along the direction indicated by a double arrow. The inflation of the balloon catheter 6 has not been started yet in FIG 7.

The radio-opaque markers 7, 8 and the longitudinal end positions 14, 15 are static and do not move. In this state, the radio-opaque markers 7, 8 and the baseline 13 may for example be identified. This may be done based on a plurality of frame intervals and respective X-ray images.

FIG 8 shows the beginning and progression of an inflation phase of the balloon catheter 6, as indicated by the double arrows. The longitudinal end positions 14, 15 remain essentially static. The positions of the baseline 13 and the lateral edges 16, 17 are computed and captured. The lateral distance between the lateral edge 16 and the baseline 13 and the lateral distance between the lateral edge 17 and the baseline 13 remain approximately equal to each other during the inflation. The diameter 18, on the other hand, increases due to the ongoing inflation until a maximum inflation state is reached. The lateral edges 16, 17 may be readjusted during the inflation.

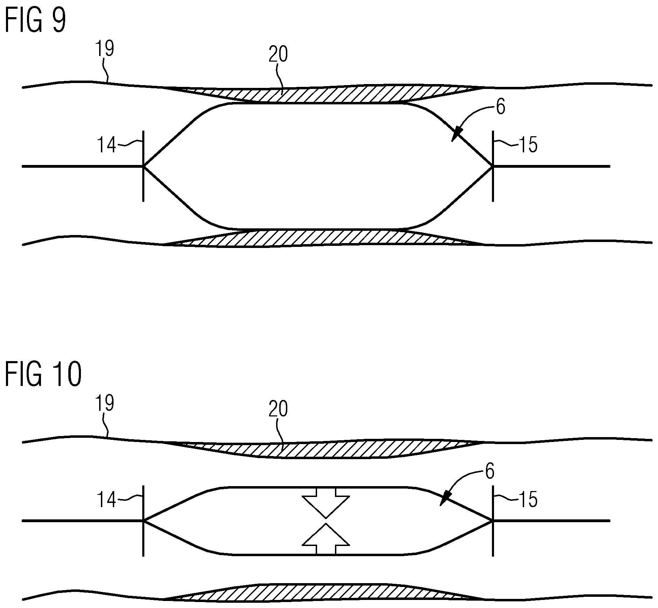

In FIG 9, the balloon catheter 6 has reached the maximum inflation state. The longitudinal end positions 14, 15 still remain essentially static and the lateral distances between the lateral edge 16, 17 and the baseline 13 also remain essentially equal to each other. The diameter 18 does not increase anymore.

FIG 10 shows the balloon catheter 6 after the deflation phase has begun. The longitudinal end positions 14, 15 still remain essentially static and the lateral distances between the lateral edge 16, 17 and the baseline 13 also remain essentially equal to each other. The diameter 18 decreases now, as indicated by the double arrows. The lateral edges 16, 17 may be readjusted during the deflation.

The inflation duration Z=Y-X may be computed as the difference between the time instance X, at which the inflation starts, and the time instance Y, at which the deflation starts. A timer may be triggered to start at the time X and may be stopped at the time Y. The inflation duration Z his duration is recorded and may be stored.

The inflation duration Z may be verified and refactored by using mathematical calculations. If there are multiple inflations and deflations, then Z may be determined as the summed inflation times of each procedure.

The inflation duration may be displayed, save and/or documented automatically without requiring a manual intervention and thus the risk of errors and effort for the staff during the procedure is reduced.

It is noted that the balloon catheter 6 may also be drug coated or act as a carrier for a stent to be deployed to the vessel 19 in some embodiments. In particular, if the blockage due to the stenosis 20 is not major, it may be possible to correct the problem by inflating the balloon catheter without deploying a stent. The inflation compacts the plaques against the vessel's 19 walls, widening the passage and improving blood flow. In case of major blockages, the stent may be deployed. It acts like a scaffold inside the vessel 19 enabling the blood flow.

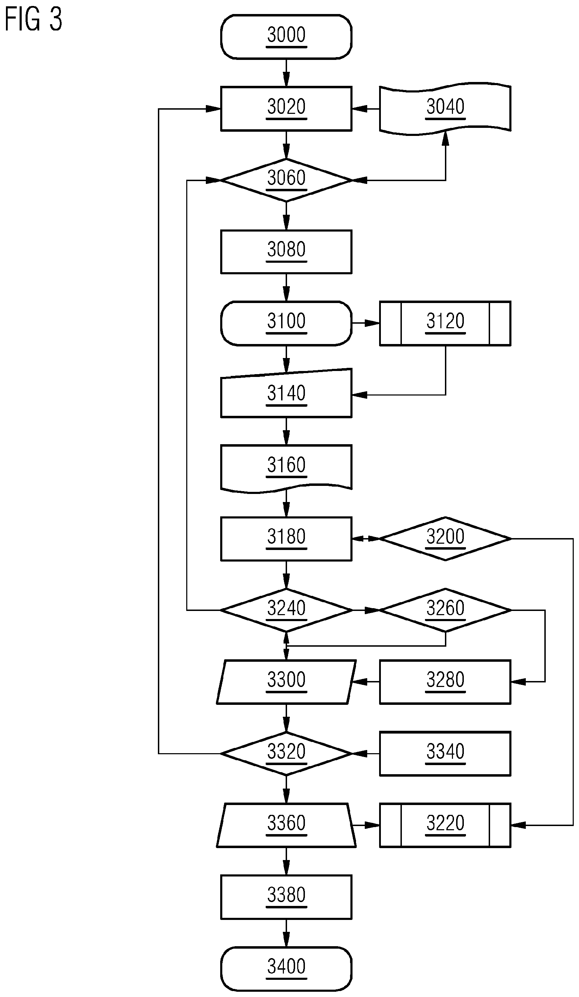

FIG 3 shows a schematic flow diagram of a medical procedure, which comprises an exemplary implementation of a method for X-ray imaging according to the invention.

The procedure starts in step 3000 with an acquisition of X-ray images. In step 3020, the stenosis 20 is located in the vessel 19. In step 3040, a mode of therapy is determined. In step 3060, it is decided to use balloon angioplasty and it is decided whether or not a stent is to be deployed. In step 3080, is decided whether or not a contrast agent is used.

In step 3100, the balloon catheter 6 is inserted into the vessel 19. In step 3210 the sequence of X-ray images depicting the balloon catheter 6 is generated. In step 3140, the balloon catheter 6 is visualized in the X-ray images. In step 3140, the balloon catheter 6 stabilizes. In step 3180, the balloon catheter's 6 geometrical parameters are determined using for example the radio-opaque markers 7, 8 and/or further image processing. In step 3200 the nominally constant geometrical parameters are monitored. In case of an inconsistency, the procedure may be interrupted and a notification to a user may be generated that the inflation duration shall be determined manually in step 3220.

In step 3240, the inflation of the balloon catheter 6 is started and the timer is started. The geometrical parameters are determined repeatedly in step 3260 and are tracked in step 3280 until the balloon catheter 6 has reached its maximum inflation state. In step 3300, the deflation of the balloon catheter 6 is started and the timer is stopped.

In step 3320 a therapy check may be performed. This may be validated in step 3340 by means of OCT and/or IVUS.

The time instances X and Y may be refactored and recorded in step 3360. The inflation duration Z is computed in step 3380 and may be displayed on a screen and/or stored in step 3400.

It is noted that the steps 3020, 3040, 3060, 3080, 3100, 3240, 3300, 3320, and 3340 are, in general, not part of the method for X-ray imaging according to the invention.

As described, in particular with reference to the figures, the accuracy and/or reliability of determining the inflation duration of a balloon catheter may be increased by means of the invention.

Various studies have determined that an inflation duration greater than 60 s on average may be favorable. Case-specific characteristics are known to affect angioplasty success, such as other systemic diseases. Other lesion characteristics also may impact the inflation duration. Prolonged inflation was associated with a reduced risk of arterial dissection and with a reduced probability that adjunctive procedures such as deploying stent become necessary. Also a long-term benefit of prolonged inflation has been reported.

The balloon deployment and optimal inflation is important also for in angioplasty procedures such as drug coated balloon percutaneous transluminal angioplasty. The balloon inflation duration has been found to be one of most critical criteria for the success of the intervention and optimal results.

Manually determining the inflation duration is error prone and cumbersome especially for the staff in the examination room. The staff may need to co-ordinate between examination rooms and control rooms to document the inflation duration and may reduce their focus on the patient and the intervention itself.

Furthermore, balloon inflation duration determined according to several implementations, whether it is one-time inflation or a multiple-time inflation MTI, may be recorded automatically, whenever a balloon catheter is used for intra-vascular interventions. This may reduce manual errors and also can be verified during a post-processing phase. Also, data collected regarding the type of procedures and their impact due to the inflation duration, can be further analyzed and made use of in future procedures to reach an optimal approach. This reduces the turnaround times and complications during intra-vascular interventions or in the post procedure phase.

There are various known methodologies and models to identify the radiopacities, for example of radio-opaque markers, guidewires and balloon shadows and other intravascular apparatus detection systems. The balloon shadows with or without contrast agents and the radio-markers can be detected by known approaches for object recognition using methods like intensity models, model-based image processing, neural networks, filtering as a detection method, in particular nonlinear filtering, linear or nonlinear discriminant analysis and so forth. The intensity of each pixel in a marker sub-image is for example given by the sum of the absorption of a standard object and the background structures along the X-rays by inclusion or exclusion criteria.

Independent of the grammatical term usage, individuals with male, female or other gender identities are included within the term.

## Claims

1. Computer implemented method for determining an inflation duration of a balloon catheter (6), wherein
- a sequence of X-ray images depicting the balloon catheter (6) inside a vessel (19) of a patient (5) is received;
- at least one geometric parameter including a diameter (18) of the balloon catheter (6) is determined based on the sequence of X-ray images and is tracked over the sequence of X-ray images;
- a first inflation starting time of a first inflation phase, during which the diameter (18) is increasing, is determined based on the tracked diameter (18);
- a first deflation starting time of a first deflation phase, during which the diameter (18) is decreasing and which lies after the first inflation phase, is determined based on the tracked diameter (18);
- the inflation duration is computed depending on a first time difference between the first inflation starting time and the first deflation starting time.

2. Computer implemented method according to claim 1, wherein the first inflation starting time is determined as a time of a transition from a first static phase, during which the diameter (18) is essentially constant, to the first inflation phase.

3. Computer implemented method according to claim 2, wherein
- the at least one geometric parameter includes a longitudinal end position of the balloon catheter (6);
- the longitudinal end position is determined to be essentially constant during the first static phase.

4. Computer implemented method according to one of the preceding claims, wherein the first deflation starting time is determined
- as a time of a transition from the first inflation phase to the first deflation phase; or
- as a time of a transition from a second static phase, during which the diameter (18) is essentially constant and which lies between the first inflation phase and the first deflation phase, to the first deflation phase.

5. Computer implemented method according to one of the preceding claims, wherein a first lateral edge position of the balloon catheter (6) and a second lateral edge position of the balloon catheter (6) are determined based on the sequence of X-ray images and the diameter (18) is determined as a distance between the first lateral edge position and the second lateral edge position.

6. Computer implemented method according to claim 5, wherein
- the at least one geometric parameter includes a lateral center position of the balloon catheter (6);
- a first lateral distance between the first lateral edge position and the lateral center position is determined and a second lateral distance between the second lateral edge position and the lateral center position is determined; and
- the first lateral distance and the second lateral distance are determined to be essentially equal during the static phase.

7. Computer implemented method according to claim 6, wherein
- a first marker position of a first radio-opaque marker (7) of the balloon catheter (6) and a second marker position of a second radio-opaque marker (8) of the balloon catheter (6) are determined based on the sequence of x-ray images;
- the lateral center position is determined depending on a straight line (13) connecting the first marker position to the second marker position.

8. Computer implemented method according to one of the preceding claims, wherein
- a second inflation starting time of a second inflation phase, during which the diameter (18) is increasing and which lies after the first deflation phase, is determined based on the tracked diameter (18);
- a second deflation starting time of a second deflation phase, during which the diameter (18) is decreasing and which lies after the second inflation phase, is determined based on the tracked diameter (18);
- the inflation duration is computed depending on a second time difference between the second inflation starting time and the second deflation starting time.

9. Computer implemented method according to claim 8, wherein the inflation duration is computed depending on a sum of the first time difference and the second time difference.

10. Data processing apparatus (2) comprising at least one computing unit, which is adapted to carry out a computer implemented method according to one of claims 1 to 9.

11. X-ray imaging device (1) comprising a data processing apparatus (2) according to claim 10, an X-ray source (4), and an X-ray detector (3), wherein
- the at least one computing unit is configured to control the X-ray source (4) to emit respective X-rays for a sequence of frame intervals;
- the X-ray detector (3) is configured to generate a respective detector dataset for each of the sequence of frame intervals depending on portions of the respective X-rays passing through a region of interest containing the vessel (19) and the balloon catheter (6) during the respective frame interval; and
- the at least one computing unit is configured to generate the sequence of X-ray images depending on the detector datasets generated for the sequence of frame intervals.

12. Computer program product comprising instructions, which, when executed by a data processing apparatus (2), cause the data processing apparatus (2) to carry out a computer implemented method according to one of claims 1 to 9.

## Patentansprüche

1. Computerimplementiertes Verfahren zum Bestimmen einer Aufblasdauer eines Ballonkatheters (6), wobei
- eine Sequenz von Röntgenbildern, die den Ballonkatheter (6) innerhalb eines Gefäßes (19) eines Patienten (5) darstellen, empfangen wird;
- mindestens ein geometrischer Parameter, der einen Durchmesser (18) des Ballonkatheters (6) beinhaltet, basierend auf der Sequenz von Röntgenbildern bestimmt und über die Sequenz von Röntgenbildern nachverfolgt wird;
- eine erste Aufblasstartzeit einer ersten Aufblasphase, während der der Durchmesser (18) zunimmt, basierend auf dem nachverfolgten Durchmesser (18) bestimmt wird;
- eine erste Ablassstartzeit einer ersten Ablassphase, während der der Durchmesser (18) abnimmt und die nach der ersten Aufblasphase liegt, basierend auf dem nachverfolgten Durchmesser (18) bestimmt wird;
- die Aufblasdauer in Abhängigkeit von einer ersten Zeitdifferenz zwischen der ersten Aufblasstartzeit und der ersten Ablassstartzeit berechnet wird.

2. Computerimplementiertes Verfahren nach Anspruch 1, wobei die erste Aufblasstartzeit als eine Zeit eines Übergangs von einer ersten statischen Phase, während der der Durchmesser (18) im Wesentlichen konstant ist, zu der ersten Aufblasphase bestimmt wird.

3. Computerimplementiertes Verfahren nach Anspruch 2, wobei
- der mindestens eine geometrische Parameter eine longitudinale Endposition des Ballonkatheters (6) beinhaltet;
- die longitudinale Endposition während der ersten statischen Phase als im Wesentlichen konstant bestimmt wird.

4. Computerimplementiertes Verfahren nach einem der vorhergehenden Ansprüche, wobei die erste Ablassstartzeit bestimmt wird
- als ein Zeitpunkt eines Übergangs von der ersten Aufblasphase zu der ersten Ablassphase; oder
- als ein Zeitpunkt eines Übergangs von einer zweiten statischen Phase, während der der Durchmesser (18) im Wesentlichen konstant ist und die zwischen der ersten Aufblasphase und der ersten Ablassphase liegt, zu der ersten Ablassphase.

5. Computerimplementiertes Verfahren nach einem der vorhergehenden Ansprüche, wobei eine erste laterale Kantenposition des Ballonkatheters (6) und eine zweite laterale Kantenposition des Ballonkatheters (6) basierend auf der Sequenz von Röntgenbildern bestimmt werden und der Durchmesser (18) als ein Abstand zwischen der ersten lateralen Kantenposition und der zweiten lateralen Kantenposition bestimmt wird.

6. Computerimplementiertes Verfahren nach Anspruch 5, wobei
- der mindestens eine geometrische Parameter eine laterale Mittelposition des Ballonkatheters (6) beinhaltet;
- ein erster lateraler Abstand zwischen der ersten lateralen Kantenposition und der lateralen Mittelposition bestimmt wird und ein zweiter lateraler Abstand zwischen der zweiten lateralen Kantenposition und der lateralen Mittelposition bestimmt wird; und
- der erste laterale Abstand und der zweite laterale Abstand während der statischen Phase als im Wesentlichen gleich bestimmt werden.

7. Computerimplementiertes Verfahren nach Anspruch 6, wobei
- eine erste Markerposition eines ersten strahlenundurchlässigen Markers (7) des Ballonkatheters (6) und eine zweite Markerposition eines zweiten strahlenundurchlässigen Markers (8) des Ballonkatheters (6) basierend auf der Sequenz von Röntgenbildern bestimmt werden;
- die laterale Mittelposition in Abhängigkeit von einer Geraden (13) bestimmt wird, die die erste Markerposition mit der zweiten Markerposition verbindet.

8. Computerimplementiertes Verfahren nach einem der vorhergehenden Ansprüche, wobei
- eine zweite Aufblasstartzeit einer zweiten Aufblasphase, während der der Durchmesser (18) zunimmt und die nach der ersten Ablassphase liegt, basierend auf dem nachverfolgten Durchmesser (18) bestimmt wird;
- eine zweite Ablassstartzeit einer zweiten Ablassphase, während der der Durchmesser (18) abnimmt und die nach der zweiten Aufblasphase liegt, basierend auf dem nachverfolgten Durchmesser (18) bestimmt wird;
- die Aufblasdauer in Abhängigkeit von einer zweiten Zeitdifferenz zwischen der zweiten Aufblasstartzeit und der zweiten Ablassstartzeit berechnet wird.

9. Computerimplementiertes Verfahren nach Anspruch 8, wobei die Aufblasdauer in Abhängigkeit von einer Summe der ersten Zeitdifferenz und der zweiten Zeitdifferenz berechnet wird.

10. Datenverarbeitungseinrichtung (2), die mindestens eine Recheneinheit umfasst, die dazu ausgelegt ist, ein computerimplementiertes Verfahren nach einem der Ansprüche 1 bis 9 durchzuführen.

11. Röntgenbildgebungsvorrichtung (1), die eine Datenverarbeitungseinrichtung (2) nach Anspruch 10, eine Röntgenstrahlquelle (4) und einen Röntgenstrahldetektor (3) umfasst, wobei
- die mindestens eine Recheneinheit dazu ausgelegt ist, die Röntgenstrahlquelle (4) zu steuern, um jeweilige Röntgenstrahlen für eine Sequenz von Bildintervallen zu emittieren;
- der Röntgenstrahldetektor (3) dazu ausgelegt ist, einen jeweiligen Detektordatensatz für jedes der Sequenz von Bildintervallen in Abhängigkeit von Teilen der jeweiligen Röntgenstrahlen zu erzeugen, die durch eine Interessensregion, die das Gefäß (19) und den Ballonkatheter (6) enthält, während des jeweiligen Bildintervalls hindurchgehen; und
- die mindestens eine Recheneinheit dazu ausgelegt ist, die Sequenz von Röntgenbildern in Abhängigkeit von den für die Sequenz von Bildintervallen erzeugten Detektordatensätzen zu erzeugen.

12. Computerprogrammprodukt, das Anweisungen umfasst, die, wenn sie durch eine Datenverarbeitungseinrichtung (2) ausgeführt werden, die Datenverarbeitungseinrichtung (2) veranlassen, ein computerimplementiertes Verfahren nach einem der Ansprüche 1 bis 9 durchzuführen.

## Revendications

1. Procédé mis en œuvre par ordinateur pour déterminer une durée de gonflage d'un cathéter (6) à ballon, dans lequel
- une séquence d'images aux rayons X décrivant le cathéter (6) à ballon à l'intérieur d'un vaisseau (19) d'un patient (5) est reçue ;
- au moins un paramètre géométrique comprenant un diamètre (18) du cathéter (6) à ballon est déterminé sur la base de la séquence d'images aux rayons X et est suivi sur la séquence d'images aux rayons X ;
- un premier temps de début du gonflage d'une première phase de gonflage, pendant laquelle le diamètre (18) est augmenté, est déterminé sur la base du diamètre (18) suivi ;
- un premier temps de début du dégonflage d'une première phase de dégonflage, pendant laquelle le diamètre (18) est diminué et qui a lieu après la première phase de gonflage, est déterminé sur la base du diamètre (18) suivi ;
- la durée de gonflage est calculée en fonction d'une première différence de temps entre le premier temps de début du gonflage et le premier temps de début du dégonflage.

2. Procédé mis en œuvre par ordinateur suivant la revendication 1, dans lequel le premier temps de début du gonflage est déterminé comme un temps d'une transition d'une première phase statique, pendant laquelle le diamètre (18) est sensiblement constant, à la première phase de gonflage.

3. Procédé mis en œuvre par ordinateur suivant la revendication 2, dans lequel
- le au moins un paramètre géométrique comprend une position d'extrémité longitudinale du cathéter (6) à ballon ;
- la position d'extrémité longitudinale est déterminée comme étant essentiellement constante pendant la première phase statique.

4. Procédé mis en œuvre par ordinateur suivant l'une des revendications précédentes, dans lequel le premier temps de début du dégonflage est déterminé
- comme un temps d'une transition de la première phase de gonflage à la première phase de dégonflage ; ou
- comme un temps d'une transition d'une deuxième phase statique, pendant laquelle le diamètre (18) est sensiblement constant et qui a lieu entre la première phase de gonflage et la première phase de dégonflage, à la première phase de dégonflage.

5. Procédé mis en œuvre par ordinateur suivant l'une des revendications précédentes, dans lequel une première position de bord latéral du cathéter (6) à ballon et une deuxième position de bord latéral du cathéter (6) à ballon sont déterminées sur la base de la séquence d'images aux rayons X et le diamètre (18) est déterminé comme distance entre la première position de bord latéral et la deuxième position de bord latéral.

6. Procédé mis en œuvre par ordinateur suivant la revendication 5, dans lequel
- le au moins un paramètre géométrique comprend une position centrale latérale du cathéter (6) à ballon ;
- une première distance latérale entre la première position de bord latéral et la position centrale latérale est déterminée et une deuxième distance latérale entre la deuxième position de bord latéral et la position centrale latérale est déterminée ; et
- la première distance latérale et la deuxième distance latérale sont déterminées comme étant essentiellement égales pendant la phase statique.

7. Procédé mis en œuvre par ordinateur suivant la revendication 6, dans lequel
- une première position de marqueur d'un premier marqueur (7) radio-opaque du cathéter (6) à ballon et une deuxième position de marqueur d'un deuxième marqueur (8) radio-opaque du cathéter (6) à ballon sont déterminées sur la base de la séquence d'images aux rayons X ;
- la position centrale latérale est déterminée en fonction d'une ligne (13) droite reliant la première position de marqueur à la deuxième position de marqueur.

8. Procédé mis en œuvre par ordinateur suivant l'une des revendications précédentes, dans lequel
- un deuxième temps de début du gonflage d'une deuxième phase de gonflage, pendant laquelle le diamètre (18) est augmenté et qui a lieu après la première phase de dégonflage, est déterminé sur la base du diamètre (18) suivi ;
- un deuxième temps de début du dégonflage d'une deuxième phase de dégonflage, pendant laquelle le diamètre (18) est diminué et qui a lieu après la deuxième phase de gonflage, est déterminé sur la base du diamètre (18) suivi ;
- la durée de gonflage est calculée en fonction d'une deuxième différence de temps entre le deuxième temps de début du gonflage et le deuxième temps de début du dégonflage.

9. Procédé mis en œuvre par ordinateur suivant la revendication 8, dans lequel la durée de gonflage est calculée en fonction d'une somme de la première différence de temps et de la deuxième différence de temps.

10. Installation (2) de traitement de données comprenant au moins une unité informatique, qui est propre à effectuer un procédé mis en œuvre par ordinateur suivant l'une quelconque des revendications 1 à 9.

11. Dispositif (1) d'imagerie par rayons X comprenant une installation (2) de traitement de données suivant la revendication 10, une source (4) de rayons X et un détecteur (3) de rayons X, dans lequel
- la au moins une unité informatique est configurée pour commander la source (4) de rayons X afin qu'elle émette des rayons X respectifs pendant une séquence d'intervalles de trame ;
- le détecteur (3) de rayons X est configuré pour créer un ensemble respectif de données de détecteur pour chacun de la séquence d'intervalles de trame, en fonction de parties des rayons X respectifs passant dans une région, à laquelle on s'intéresse, contenant le vaisseau (19) et le cathéter (6) à ballon pendant l'intervalle de trame respectif ; et
- la au moins une unité informatique est configurée pour créer la séquence d'images aux rayons X, en fonction des ensembles de données de détecteur créés pendant la séquence d'intervalles de trame.

12. Produit de programme d'ordinateur comprenant des instructions, qui, lorsqu'elles sont exécutées par une installation (2) de traitement de données, font que l'installation (2) de traitement de données exécute un procédé mis en œuvre par ordinateur suivant l'une des revendications 1 à 9.
